## Europäisches Patentamt
## European Patent Office
## Office européen des brevets

(19)

(11) Publication number: **0 082 187**
**B1**

(12)

# EUROPEAN PATENT SPECIFICATION

(45) Date of publication of patent specification: **07.10.87**

(51) Int. Cl.⁴: **A 61 M 1/34,** G 05 D 11/00

(21) Application number: **82902285.4**

(22) Date of filing: **04.06.82**

(86) International application number:
**PCT/US82/00771**

(87) International publication number:
**WO 83/00020 06.01.83 Gazette 83/01**

(54) **MEMBRANE PLASMAPHERESIS APPARATUS.**

(30) Priority: **25.06.81 US 277428**
**25.06.81 US 277449**

(43) Date of publication of application:
**29.06.83 Bulletin 83/26**

(45) Publication of the grant of the patent:
**07.10.87 Bulletin 87/41**

(84) Designated Contracting States:
**BE CH DE FR GB LI SE**

(56) References cited:
US-A-3 693 611
US-A-3 795 318
US-A-3 918 677
US-A-4 113 614
US-A-4 191 182

**Trans. American Society for Artificial Internal
Organs, Vol. 14, 4/1978, Solomon et al.:
"Continuous Flow Membrane Filtration of
Plasma from Whole Blood"**

(73) Proprietor: **BAXTER TRAVENOL
LABORATORIES, INC.
One Baxter Parkway
Deerfield, IL 60015 (US)**

(72) Inventor: **KOPP, Clinton V.
204 Terrace Drive Tower Lake
Barrington, IL 60015 (US)**
Inventor: **HITCHCOCK, James
435 North Avenue
Barrington, IL 60010 (US)**

(74) Representative: **MacGregor, Gordon et al
ERIC POTTER & CLARKSON 14 Oxford Street
Nottingham, NG1 5BP (GB)**

Courier Press, Leamington Spa, England.

## Description

### Field of the Invention

This invention generally relates to membrane plasmapheresis apparatus.

### Description of the Prior Art

During the process of membrane plasmapheresis, plasma is removed from whole blood by filtration through a microporous membrane. In order for plasma to pass through the membrane, a driving force is required. This force is known as the transmembrane pressure.

When the transmembrane pressure is at an operationally desirable level, the blood flows across the membrane with a sufficient velocity and sheer rate to cause only plasma to pass through the pores of the membrane for collection. However, should the transmembrane pressure rise above a certain critical level, the red cells can themselves be forced into the membrane pores and be hemolyzed.

Effecting the magnitude of the transmembrane pressure is the inlet pressure of the whole blood, as well as the ressistance the whole blood encounters as it travels across the membrane. Also contributing to the magnitude of the transmembrane pressure is the resistance the plasma-poor blood encounters as it is being returned to the patient-donor through an associated transfusion set, phlebotomy needle, blood warmer and other ancillary apparatus located downstream of the membrane. The blood pressure of the individual patient-donor and movement of his or her arm during the course of the procedure also serve to increase this downstream resistance, which can and typically does fluctuate suddenly and randomly.

These sudden and random fluctuations in the resistance to the flow of plasma-poor blood can elevate the transmembrane pressure to a point where hemolysis occurs.

In the past, efforts to minimize the magnitude of the downstream resistance encountered during membrane plasmapheresis, and thus to stabilize the transmembrane pressure at operationally desirable levels, have included the use of a larger needle, which can, of course, contribute to patient discomfort; forgoing the use of ancillary equipment downstream of the membrane, even through operationally desirable; and continuously changing the elevation of the plasma filtrate collection container during the procedure. These efforts are, at best, stopgap measures and often run counter to the overall objectives of a comfortable and efficient membrane plasmapheresis operation.

Attention is also directed to US—A—4,191,182. In the plasmapheresis procedure therein disclosed, the plasma filtrate is constantly recirculated over the filtering side of the membrane to stabilize the transmembrane pressure across the length of the membrane. However, such a procedure fails to address the problems associated with random and sudden pressure fluctuations occurring downstream of the membrane.

Objects of this invention are to provide a membrane plasmapheresis system in which the transmembrane pressure is maintained at a stable and constant magnitude below the level at which hemolysis occurs, in which the magnitude of the transmembrane pressure is subject to the constant control of the operator and does not experience sudden and random fluctuations, which permits both the comfort of the patient-donor as well as the operational efficiency of the procedure itself to be maximized to the fullest extent possible.

The present invention provides a membrane plasmapheresis apparatus as defined in claim 1, in which the pre-characterising clause is based on US—A—4,191,182 and the characterising clause defines the distinguishing features of the present invention.

The invention thus provides a membrane plasmapheresis apparatus having a constant and stable transmembrane pressure which is subject to control by the operator and which is uneffected by sudden and random fluctuations in the plasma-poor blood pressure downstream of the membrane. The invention also provides a membrane plasmapheresis apparatus in which an uninterrupted flow of plasma filtrate and plasma-poor blood occurs downstream of the module. At the same time, the invention permits the use of ancillary equipment downstream of the membrane to maximize the operational efficiency of the membrane plasmapheresis procedure, without the attendant fear of elevating the transmembrane pressure above a point where hemolysis can occur.

The invention also provides a membrane plasmapheresis apparatus comprising a fluid flow control device which comprises first conduit means adapted for communication with a first source of pressurized fluid and second conduit means adapted for communication with a second source of pressurized fluid. The device further includes third conduit means in flow communication with the first conduit means for conducting the pressurized fluid from the first conduit means. The third conduit means includes generally flexible first wall means forming an interface with a portion of the second conduit means. The first wall means is operative in response to fluid pressures in the second conduit means for metering the flow communication between the first and third conduit means. The metering action of the flexible first wall means adjusts the fluid pressure in the first conduit means until it achieves substantial equilibrium with the fluid pressure than existent in the second conduit means, and thereafter maintains this state of substantial equilibrium, notwithstanding subsequent fluctuations in the pressure in the second conduit means.

In accordance with one embodiment, the device includes means for maintaining constant flow communication between the first and third fluid conduit means, despite the continuous metering action of the flexible first wall means.

## Description of the Drawings

Fig. 1 is an essentially diagrammatic view of a membrane plasmapheresis apparatus which embodies various of the features of the invention and which utilizes a module in which spaced-apart sheets of microporous membranes are supported;

Fig. 2 is an essentially diagrammatic view of a module in which a cluster of hollow fiber membranes are supported and which is adapted for use with the membrane plasmapheresis apparatus shown in Fig. 1;

Fig. 3 is an enlarged, diagrammatic and section view of a fluid flow control device suited for use with the apparatus shown in Fig. 1;

Fig. 4 is a perspective view of one structural embodiment of the pressurized fluid flow control device shown in Fig. 3;

Fig. 5 is an exploded perspective view of the device shown in Fig. 4, with parts broken away to show the flow of fluids through the device; and

Fig. 6 is another exploded perspective view of the component parts of the device shown in Fig. 4.

## Description of the Preferred Embodiment

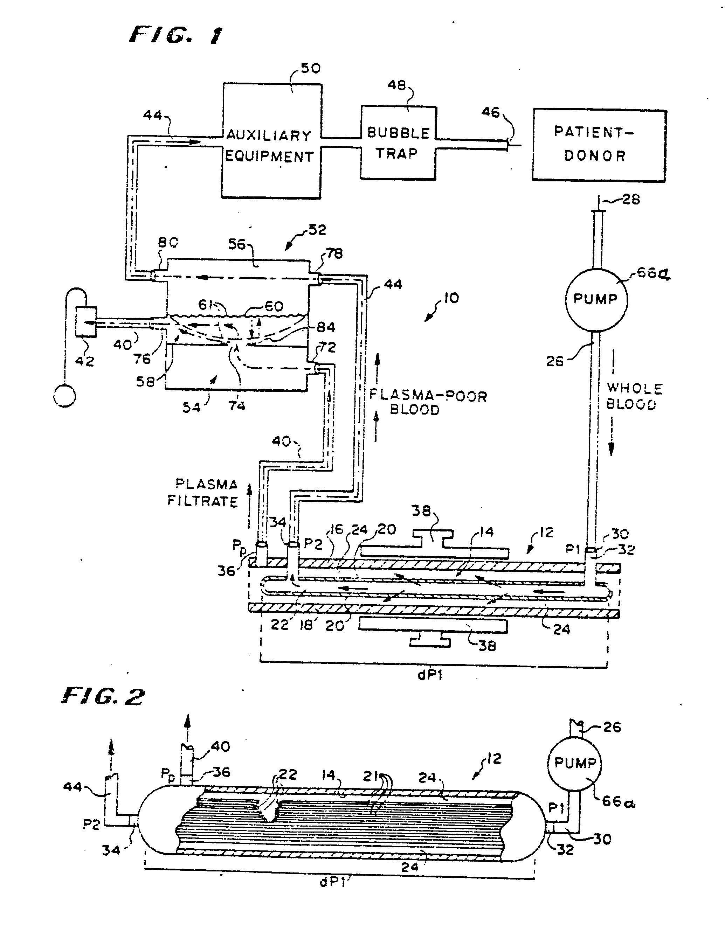

A membrane plasmapheresis apparatus 10 is shown in Fig. 1 which is operative for removing, or "harvesting", the plasma from whole blood for exchange, transfusion, or fractionation purposes.

The apparatus 10 includes a module or cell 12 in which microporous membranes 14 are housed. Various membranes 14 can be used, provided that they have a pore size suited for separating the plasma from whole blood, given the proper conditions of pressure and flow rates across the membranes surfaces.

The module 12 itself may also be variously constructed, according to the membrane configuration utilized. Two alternate embodiments are shown, respectively, in Figs. 1 and 2. It should be appreciated, however, that numerous other embodiments are possible.

In the embodiment shown in Fig. 1, the module 12 includes first and second generally planar members, respectively 16 and 18, defining a housing in which two sheets of a microporous membrane 20 having a pore size of about 0.1 micron to 2 microns are positioned in a facing, spaced-apart relationship. A fluid path 22 is thus formed between the membranes 20, and the module 12 includes an inlet and outlet port, respectively 32 and 34, communicating at opposite ends of the fluid path 22. Open volumes 24 are also formed between the outer surfaces of the membranes 20 and the interior surfaces of the planar members 16 and 18, and the module 12 includes an outlet port 36 communicating with the volumes 24.

In the alternative embodiment shown in Fig. 2, the module 12 takes the form of a generally tubular housing in which a cluster of individual hollow fiber membranes 21 is supported. The hollow fiber membranes 21 can be fabricated from various materials, for example, polypropylene having a pore size of about 0.6 microns and an average pore size in the neighborhood of 0.3 microns. The hollow bore of each fiber 21 defines the fluid path 22 corresponding to the one heretofore described with respect to the Fig. 1 embodiment. The tubular housing is constructed so as to form an open volume 24 circumferentially enveloping the hollow fiber cluster. As in the Fig. 1 embodiment, the module 12 includes inlet port 32 and outlet ports 34 and 36.

The apparatus 10 also includes a whole blood inlet conduit 26 having at one end thereof a phlebotomy needle 28 for insertion into the arm of a patient-donor. The other end 30 of the conduit 26 is attached to the inlet port 32 of the module 12.

The apparatus 10 further includes an in-line pump 66a, for example, a peristaltic pump, which delivers whole blood from the patient-donor to the inlet port 32 of the module 12 subject to a predetermined inlet pressure will hereafter be referred to and is shown in Figs. 1 and 2 as P1.

As the whole blood traverses the length of the fluid pathway 22, a predetermined pressure drop occurs. This pressure drop is symbolized as dP1 in Figs. 1 and 2. The size of the pressure drop dP1 depends principally upon the fluid volume of the pathway 22, which, in the Fig. 1 embodiment, depends in large part upon the lateral spacing between the sheet membranes 20. This lateral spacing is controlled by use of an adjustable clamp 38, which presses the planar members 16 and 18 together to achieve the desired pressure drop dP1.

In the Fig. 2 embodiment, the interior diameter of the hollow bore of each hollow fiber 21 is preselected to achieve the desired pressure drop, obviating the need for the clamp 38.

By carefully controlling the magnitude of the inlet pressure P1 and the pressure drop dP1, the whole blood experiences a desired sheer rate as it traverses the membranes 20 or 21. This causes the red cells, leukocytes, and platelets to proceed in a laminar path across the membranes 20 or 21. At the same time, a determinable transmembrane pressure, or TMP, is generated, which, when within the operationally desirable limits of between approximately 50 mmHg to 100 mmHg (0.067 to 0.133 bars) acts as a driving force to cause only the plasma to pass through the pores of the membranes 20 or 21 and into the volumes 24. However, as will be discussed in greater detail later herein, should the transmembrane pressure exceed a critical level (approximately 120 mmHg (0.160 bars)), hemolysis can occur.

To conduct the plasma filtrate from the module 12, the apparatus 10 includes a collection conduit 40 attached to the outlet port 36 in flow communication with the plasma filtrate volumes 24. The conduit 40 has an end connected to a plasma filtrate collection bag 42. Typically, this plasma filtrate is subject to a pressure (Pp in Figs. 1 and 2) and which is at or near atmospheric pressure or 0 mmHg.

To conduct the cellular components which do not pass through the membranes 20 or 21 from the module 12, these components now being collectively referred to as plasma-poor blood, the apparatus 10 includes a transfusion set 44 attached to the outlet port 34. The transfusion set 44 includes a phlebotomy needle 46 for insertion into the patient-donor to return the conducted plasma-poor blood to the patient-donor.

A bubble trap 48 is preferably connected in line with the transfusion set 44 between the outlet port 34 and the needle 46. Auxiliary equipment (generally designated by the numeral 50 in Fig. 1), such as a blood warmer, can also be connected in line with the transfusion set 44 to enhance the conduction of plasma-poor blood back to the patient-donor.

It has been observed that the presence of the various operationally necessary or desirable equipment positioned in the flow path of the plasma-poor blood downstream of the module 12 (i.e., the transfusion set 44, the needle 46, and any auxiliary equipment 50) collectively generates a resistance to the return of the plasma-poor blood. This resistance will hereafter be identified as the backside pressure (symbolized as P2 in Figs. 1 and 2). Also contributing to the magnitude of the backside pressure P2 is the blood pressure of the individual patient-donor, as well as any random movement of patient-donor's arm during the procedure, which can cause a temporary occlusion in the flow path. The magnitude of the backside pressure P2 is often significant and can suddenly and randomly fluctuate during the course of the procedure between 20 mmHg and 150 mmHg (0.027 and 0.200 bars).

It has also been observed that these sudden and random fluctuations in the backside pressure P2, if not compensated for, serve to induce correspondingly sudden and random variations in the transmembrane pressure of the apparatus 10. Thus, the presence of equipment downstream of the module 12 causes the transmembrane pressure to be unstable and can lead to sudden and random elevations of the transmembrane pressure above operationally desirable levels to a magnitude above 120 mmHg (0.160 bars). At this critical level, the red cells traversing the membranes 20 or 21 can themselves be driven into the pores of the membranes 20 or 21 and be torn, damaged, or destroyed. Hemolysis results.

Efforts can be made to establish the transmembrane pressure at operationally desirable levels by removing as many of the sources of the backside pressure P2 as possible. For example, the size of the phlebotomy needle 46 can be enlarged (the smaller the needle, the larger the pressure developed, and vice versa), but this, in turn, can lead to patient-donor discomfort. Or, the use of ancillary equipment 50 downstream of the module 12 can be minimized, but such equipment is desirable for an efficient plasmapheresis procedure. To deal with the problem, the operator can also constantly adjust the elevation of the plasma collection bag 42, but such activities divert operator attention from other necessary duties. In short, efforts such as those detailed run counter to patient-donor comfort and an efficient membrane plasmapheresis procedure and indeed do not and cannot completely stabilize the transmembrane pressure. The blood pressure of the particular patient-donor, or any arm movement of the patient-donor during the procedure, are variables which simply cannot be anticipated and instantly compensated for.

In order to effectively stabilize the transmembrane pressure at operationally desired levels, regardless of the presence of and fluctuations in the backside pressure P2, the apparatus 10 includes fluid flow control means 52. The control means 52 is operative for metering the flow of plasma filtrate exiting the volumes 24 in response to the then existent backside pressure P2 to maintain the pressure of the plasma filtrate (or Pp) at a magnitude equal to the magnitude of the then existent backside pressure P2.

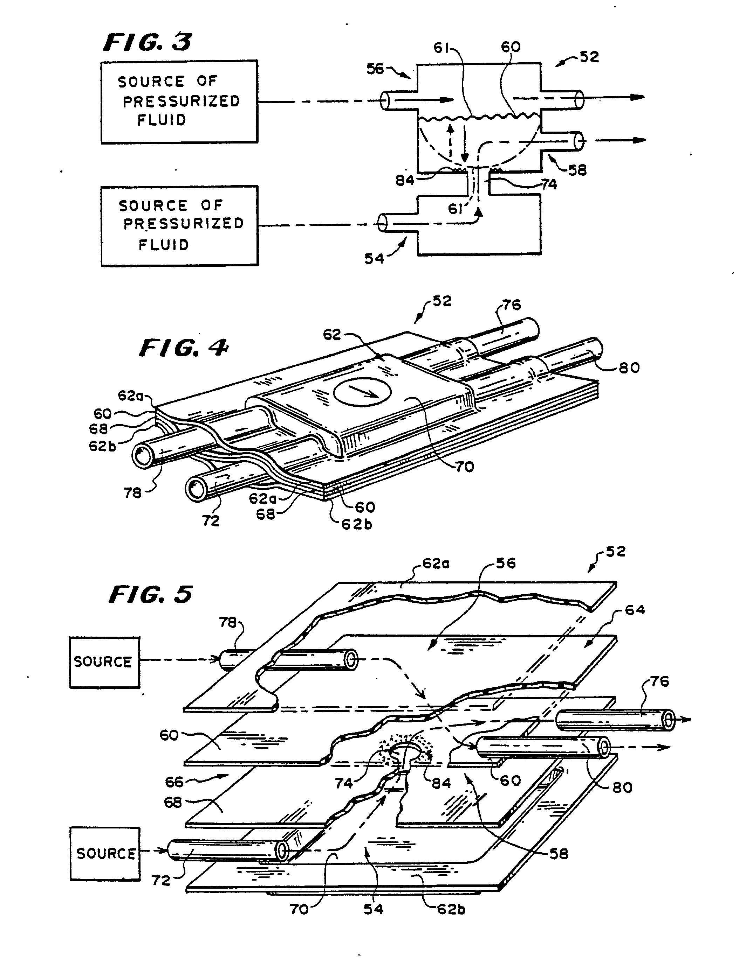

The control means 52 can be variously constructed. In the illustrated embodiment, as shown in Fig. 3, the control means 52 includes conduit means defining first, second and third fluid pathways, respectively 54, 56 and 58. The first and second fluid pathways 54 and 56 are each individually adapted to communicate with its own source of pressurized fluid for conducting fluid from the respective source. The third fluid pathway 58 is in flow communication with the first fluid pathway 54 to conduct pressurized fluid therefrom.

In addition, the third fluid pathway 58 includes first wall means 60 which forms an interface with a portion of the second fluid pathway 56. The first wall means 60 is made of a flexible material and is operative for movement (as is generally shown by the use of arrows and phantom lines in Fig. 3) to meter the flow communication between the first and third fluid pathways 54 and 58.

More particularly, and still referring principally to Fig. 3, in response to an initial condition in which the fluid pressure in the second pathway 56 exceeds the fluid pressure in the first pathway 54, the first wall means 60 is moved in response to the pressure differential to restrict the flow of fluid between the first and third pathways 54 and 58. This serves to elevate the fluid pressure in the first pathway 54 until substantial equilibrium between the fluid pressures in the first and second pathways 54 and 56 occurs. Thereafter, the first wall means 60 is operative to maintain this condition of substantial equilibrium, notwithstanding any subsequent variations in the fluid pressure in the second pathway 56.

The control means 52 as heretofore generally described may be assembled in various ways. In the particular embodiment shown in Figs. 4 through 6, the control means 52 comprises a compact housing 62 enclosing an interior area 64. The interior area 64 is itself compartmentalized by spaced wall means 66 into the three fluid pathways 54, 56 and 58 heretofore described.

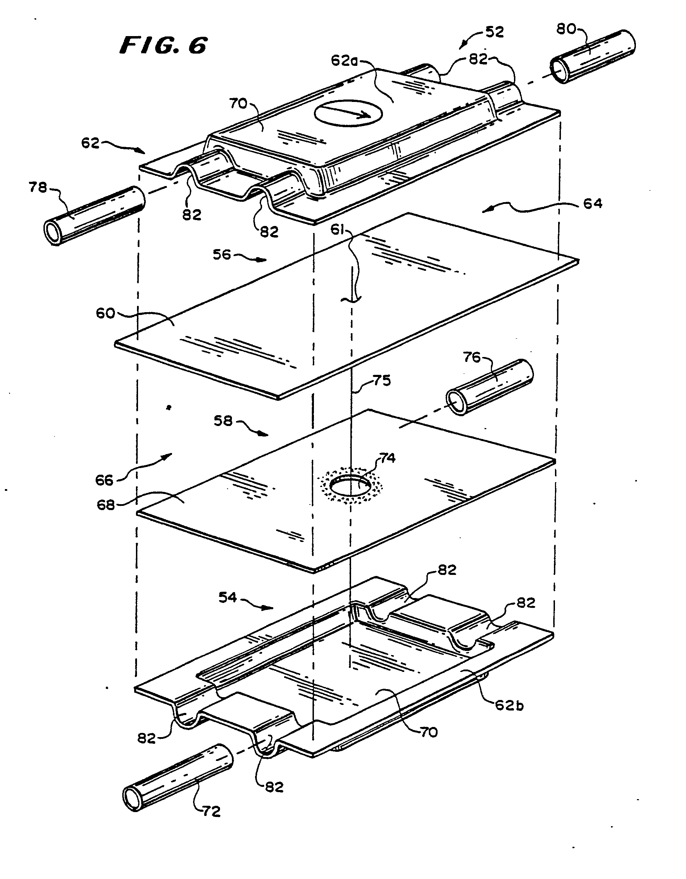

More particularly, and as best seen in Figs. 5 and 6, the wall means 68 includes the previously described first wall means 60 which forms the flexible interface between the second and third fluid pathways 56 and 58. The wall means 66 also includes second wall means 68, which forms an interface between the first and third fluid pathways 54 and 58.

In this arrangement, as can best be seen in Fig. 5, the three fluid pathways 54, 56 and 58 extend in a generally parallel and stacked relationship one above the other within the housing interior 64. The third fluid pathway 58 is positioned in this arrangement between the first and second pathways 54 and 58, which are themselves spaced at opposite ends of the interior area 64. By virtue of the raised exterior portions 70 of the housing 62, the enclosed portions of the first and second pathways 54 and 56 can be viewed as defining oppositely spaced chambers within the housing interior 64.

In this assemblage, the first pathway 54 includes an inlet portion 72 which extends outwardly of the housing 62 and which is adapted for communication with one source of pressurized fluid. The second wall means 68 is provided with an opening 74 which, in the illustrated embodiment, extends generally at a right angle to the fluid flow path through the first pathway 54 and which provides the heretofore described flow communication between the first and third fluid pathways 54 and 58. In this respect, the opening 74 serves as an outlet portion for the first fluid pathway 54 and an inlet portion for the third fluid pathway 58. The third fluid pathway 58 further includes an outlet portion 76 which extends outwardly of the housing 62 and which communicates with the atmosphere.

The second fluid pathway 56 includes spaced, generally coplanar inlet and outlet portions, respectively 78 and 80, both of which extend outwardly of the housing 62. The inlet portion 78 is adapted for communication with the other source of pressurized fluid, and the outlet portion 80 communicates with the atmosphere.

This compact structural arrangement lends itself to construction utilizing relatively few preformed parts. It also lends itself to construction utilizing only plastic materials and the like which have been approved for contact with human blood. As a result, the illustrated embodiment of the control means 52 can be manufactured in an efficient and economical manner and constitute an essentially disposable unit.

More particularly, in the illustrated embodiment (see Fig. 6), the housing 62 includes upper and lower housing portions, respectively 62a and 62b, which can be manufactured from a suitable plastic material, such as by the use of injection molding techniques. Preferably, the upper and lower housing portions 62a and 62b are generally rigid or semirigid in construction and include a spaced pair of outwardly bowed or convex grooves 82 formed at each opposite end.

The second wall means 68 constitutes a presized sheet of suitable generally flexible plastic material in which the opening 74 is centrally located. The first wall means 60 comprises a presized sheet of flexible plastic material, with a thickness of approximately 15 mils.

To form the desired compartmentalization within the housing 62 (and as best seen in Fig. 5), the inlet portion 72 of the first fluid pathway 54 comprises a presized section of plastic polyvinyl chloride tubing which is sandwiched between the lower housing portion 62b and the second wall means 68. The grooves 82 of the upper and lower housing portions 62a and 62b form a bushing to receive the inlet tubing portion 72 (see Fig. 4).

The inlet and outlet portions 78 and 80 of the second fluid pathway 56 likewise comprise identical presized sections of plastic polyvinyl chloride tubing positioned diagonally across from each other and sandwiched between the upper housing portion 62a and the flexible sheet comprising the first wall means 60. This diagonal relationship between the tubing portions 78 and 80 assures a uniform, laminar flow of fluid within the second fluid path 56.

As before, the grooves 82 of the upper and lower housing portions 62a and 62b together form bushings to receive the inlet and outlet tubing portions 78 and 80.

In similar fashion, the outlet portion 76 of the third fluid pathway 58 comprises a presized section of plastic polyvinyl chloride tubing which is positioned generally diagonally across from the inlet tubing portion 72 of the first fluid pathway 54 and there sandwiched between the first wall means 60 and the second wall means 68 within the bushing formed by the cooperating grooves 82. The diagonal relationship between the tubing portions 72 and 76 assures a uniform and laminar flow of fluid into and out of the first and third fluid pathways 54 and 58 through the interconnecting opening 74.

The entire assembly shown in Figs. 5 and 6 is sandwiched together into the compact configuration shown in Fig. 4 and peripherally sealed, such as by the use of radio frequency, heat, or solvent sealing methods. During such manufacture, the peripheries of the flexible first and second wall means 60 and 68 will conform to sealingly surround the adjacent tubing portions.

As can be best seen in Figs. 5 and 6, the flexible first wall means 60 is generally oppositely spaced in facing relationship from the opening 74 formed in the second wall means 68. Furthermore, the axis 75 of the opening 74 (see Fig. 6) is generally aligned with the midportion 61 of the first wall means 60. Because of this construction, coupled with the inherent flexibility of the first wall means 60, the first wall means 60 is operative for movement in response to fluid pressures in a path along the axis 75 toward and away from the opening 74.

While the control means 52 of the above described construction can be of various sizes, in one operative embodiment thereof, the housing 62 is approximately 2.5 inches (64 mm), in overall length (exclusive of the outwardly extending tubings), approximately 1.5 inches (38 mm) in overall width, and approximately 0.5 inches (13 mm) in overall thickness.

Referring now back to Fig. 1, the control means 52 is connected downstream of the module 12 in flow communication with both the transfusion set 44 and the plasma collection conduit 40. More particularly, the inlet portion 72 of the first fluid pathway 54 is attached in flow communication with the outlet port 36, and the outlet portion 76 of the third fluid pathway 58 is attached in flow communication with the plasma collection container 42.

As a result of this interconnection in line with the collection conduit 40, plasma filtrate flows out of the plasma volumes 24, subject to the plasma pressure Pp, into and through the first and third fluid pathways 54 and 58 via the opening 74, and thence toward the plasma collection container 42.

The inlet and outlet portions 78 and 80 of the second fluid pathway 56 are connected in line with transfusion set 44 upstream of the bubble trap 48 and any associated auxiliary equipment 50.

As a result of this in line connection, the plasma-poor blood flows from the outlet port 70 subject to the back pressure P2, into and through the laminar flow path of the second fluid pathway 56, and thence toward the downstream equipment. The laminar flow path provided by the second fluid pathway 56 minimized undesirable mixing, or turbulence, of the plasma-poor blood during its return to the patient-donor. This, in turn, reduced the chance of hemolysis which can be occasioned by such mixing.

At the outset of the plasmapheresis procedure, a pressure differential will always exist between the plasma pressure Pp (typically at 0 mmHg) and the backside blood pressure P2 (typically between 20 mmHg and 150 mmHg (0.027 and 0.200 bars)). Assuming that the flexible first wall means 60 is disposed in an initial position (shown in solid lines in Figs. 1 and 3) which is generally parallel with the second wall means 68, the flexible first wall means 60 will respond to the pressure differential by moving in its axial path toward the opening 74. This is generally shown by arrows and in phantom lines in Figs. 1 and 3.

This movement of the flexible wall means 60 toward the opening 74 will result in a change in its configuration of the flexible first wall means 60 from its essentially planar initial position (shown in solid lines in Figs. 1 and 3) toward a generally convex configuration outwardly bowed into the third fluid pathway 58 (shown in phantom lines in Figs. 1 and 3).

Closer proximity of the flexible first wall means 60, and, in particular, its midportion 61, to the opening 74 serves to restrict the flow of plasma filtrate through the opening 74. This restriction, in turn, causes the plasma fluid pressure Pp in the first pathway 54 to rise. This plasma pressure elevation will proceed until pressure equalization with the then existent backside fluid pressure P2 in the second pathway 56 occurs.

It should be appreciated that the particular configuration the flexible wall means 60 will assume within the third pathway 58 and relative to the opening 74 to meter the flow of plasma filtrate and bring about pressure equalization will depend upon the particular magnitudes of the then prevailing plasma filtrate and plasma-poor blood pressures Pp and P2, as well as the then prevailing fluid flow rates.

Should the backside pressure P2 in the second pathway 56 subsequently increase or decrease, the flexible wall means 60 will correspondingly change its configuration by moving in its axial path toward a new position, respectively, closer to or farther away from the opening 74. This automatically changes the previously imposed restriction to the flow of plasma filtrate in lieu of a new restriction. The flow of plasma filtrate will be metered at the new rate until the plasma filtrate pressure Pp achieves equalization with the higher or lower backside pressure P2.

In reality, the flexible first wall means 60 is thus movable in its axial path through a range of positions which are progressively spaced closer to or farther away from the opening 74. The particular position and configuration of the flexible wall means 60 within this range will depend upon the particular fluid pressures and flow rates then prevailing.

It should be appreciated that the movement of the flexible wall means 60 as just described occurs virtually instantaneously with fluctuations in the backside pressure P2. Thus, the control means 52 is operative to continuously maintain pressure equilibrium between the plasma filtrate pressure Pp and the backside pressure P2.

It also be appreciated that the control means 52 as heretofore described preferably operates without the use of valve seats or the like. Thus, the movement of the flexible wall means 60 toward the opening 74 will not normally serve to completely close or seal the opening 74, and thereby completely block flow communication therethrough.

Furthermore, in the illustrated and preferred embodiment, the area 84 circumferentially surrounding the opening 74 is roughened or contoured to break any surface tension that might develop between the area 84 and the flexible wall means 60. This further assures that the flexible wall means 60 will not assume a position completely blocking the opening 74, thereby assuring a desirable constant and continuous flow of plasma filtrate in the apparatus 10.

The plasmapheresis apparatus 10 as above described has been observed to continuously meter the conduction of plasma filtrate in response to the plasma-poor, or backside, blood pressure P2 to maintain substantial equilibrium between the pressure of the plasma filtrate (Pp) and the backside pressure P2.

The resulting transmembrane pressure of the apparatus 10 has been observed to be essentially stable, regardless of the magnitude of the then existent backside pressure P2, at a magnitude which represents only the flow resistance of the module 12 itself (or P1—dP1), which is a quantity under direct operator control. In particular, when an inlet pressure P1 of between 150 mmHg and 200 mmHg (0.200 and 0.267 bars) is maintained, along with a constantly maintained pressure drop Dpl across the module 12 of approximately 100 mmHg (0.133 bars), the apparatus 10 serves to stabilize the transmembrane pressure within the operationally desirable range of between 50 mmHg (0.067 bars) and 100 mmHg (0.133 bars), even through the backside pressure P2 may at the same time be undergoing random fluctuations of between 200 mmHg and 150 mmHg (0.267 and 0.200 bars).

In addition, the apparatus 10 has been observed to continuously maintain an uninterrupted flow of plasma filtrate from the module 12 and through the first and third pathways 54 and 58 at a rate of between 10 cubic centimeters per minute and 80 cubic centimeters per minute. At the same time, the apparatus 10 has been observed to continuously maintain an uninterrupted flow of plasma-poor blood from the module 12 and through the second pathway 56 at a rate of between 40 cubic centimeters per minute and 300 cubic centimeters per minute.

The apparatus 10 also permits the use of operationally desirable components of membrane plasmapheresis, such as a smaller, more comfortable needle, and auxiliary equipment such as the blood warmer, without effecting the stability of the transmembrane pressure and without causing hemolysis.

**Claims**

1. A membrane plasmapheresis apparatus comprising membrane means (20; 21) for filtering the plasma from whole blood, means (22, 24) for forming a fluid path across said membrane means subject to a predetermined pressure drop, means (32) communicating with said membrane means and adapted for communication with a source of whole blood for introducing the whole blood subject to a predetermined inlet pressure into said fluid path to filter the plasma from the whole blood, means (34, 44, 56) communicating with said membrane means for conducting plasma-poor blood from said fluid path subject to a plasma-poor blood pressure which is subject to random variations, means (36, 40, 54, 58) communicating with said membrane means for conducting plasma filtrate from said fluid path (24) subject to a plasma filtrate pressure, and equaliser means (52) to control the pressure drop across said membrane means and to adjust the transmembrane pressure, characterised in that the equaliser means comprises a flexible interface (60) between a portion (56) of said plasma-poor blood conduction means and a portion (58) of said plasma filtrate conduction means for variably restricting the flow of plasma filtrate through said plasma filtrate conduction means in response to the pressure differential between said plasma-poor blood conduction means and said plasma filtrate conduction means to establish and thereafter maintain substantial equilibrium between the pressure of the plasma filtrate and the pressure of the plasma-poor blood, despite random variations in the plasma-poor blood pressure.

2. A membrane plasmapheresis apparatus according to Claim 1 wherein said equaliser means maintains an uninterrupted flow of plasma filtrate through said portion (58) of the plasma filtrate conduction means.

3. A membrane plasmapheresis apparatus according to Claim 2, wherein the equaliser means includes means (60, 68, 62, 72, 76) for maintaining a laminar flow through said portion (58) of the plasma filtrate conduction means.

4. A membrane plasmapheresis apparatus according to Claim 1, 2 or 3 wherein said equaliser means maintains an uninterrupted flow through said portion (56) of the plasma-poor blood conduction means.

5. A membrane plasmapheresis apparatus according to Claim 4 wherein said equaliser means includes means (60, 62, 78, 80), for maintaining a laminar flow through said portion (56) of the plasma-poor blood conduction means.

6. A membrane plasmapheresis apparatus according to Claim 1, 2, 3, 4 or 5 wherein said equaliser means includes a first conduit portion (54) forming a portion of said plasma filtrate conduction means, a second conduit portion (56) forming a portion of said plasma-poor blood conduction means, and a third conduit portion (58) communicating with said first conduit portion (54) and including a flexible first wall (60) which forms said flexible interface and which is operative in response to fluid pressures in said plasma filtrate conduction means and said plasma-poor blood conduction means for metering fluid flow between said first (54) and third (58) conduit portions to establish and thereafter maintain said substantial equilibrium between fluid pressures in said plasma filtrate and plasma-poor blood conduction means.

7. A membrane plasmapheresis apparatus according to Claim 6 wherein said first conduit portion (54) defines a first chamber, said second conduit portion (56) defines a second chamber, the flexible first wall (60) forms an interface with the second chamber, and the third conduit portion (58) includes a second wall (68) forming an interface with the first chamber and having an opening (74) establishing flow communication between the first chamber and the third conduit portion.

8. A membrane plasmapheresis apparatus according to Claim 7 wherein the first wall (60) is generally oppositely spaced from the second wall (68) and is operative for movement, in response to fluctuations in the fluid pressures in said second chamber (56), in a path axially towards and away from said opening (74) to meter flow communication therethrough.

9. A membrane plasmapheresis apparatus according to Claim 8 wherein, in response to an increase in the fluid pressures in said second chamber (56), the flexible first wall (60) is movable in said path towards said opening (74) so as increasingly to restrict flow communication therethrough, and wherein, in response to a decrease in said fluid pressures in the second chamber (56), the flexible first wall (60) is movable in said path away from said opening to diminish the restriction of said flow communication.

10. A membrane plasmapheresis apparatus according to Claim 9 wherein the construction is such that continuous flow communication between the first (54) and third (58) conduit means is maintained regardless of metering by the flexible first wall (60).

**Patentansprüche**

1.     Membran-Plasmaphereservorrichtung umfassend einen Membrankörper (20; 21) zum Herausfiltern von Plasma aus Vollblut, Mittel (22, 24) zur Bildung eines Fluidwegs über den Membrankörper in Abhängigkeit von einem vorbestimmten Druckabfall, mit dem Membrankörper in Verbindung stehende Mittel (32), die mit einer Vollblutquelle verbindbar sind zur Einleitung des Vollbluts in Abhängigkeit von einem vorbestimmten Einlaßdruck in den Fluidweg zum Herausfiltern des Plasmas aus dem Vollblut, mit dem Membrankörper in Verbindung stehende Mittel (34, 44, 56), die plasmaarmes Blut in Abhängigkeit von einem von zufälligen Änderungen abhängigen Druck plasmaarmen Blutes aus dem Fluidweg leiten, mit dem Membrankörper in Verbindung stehende Mittel (36, 40, 54, 58), die Plasmafiltrat in Abhängigkeit von einem Plasmafiltrat-Druck aus dem Fluidweg (24) leiten, und Ausgleichsmittel (52) zum Steuern des Druckabfalls über den Membrankörper und zum Einstellen des Transmembrandrucks, dadurch gekennzeichnet, daß die Ausgleichsmittel eine flexible Grenzfläche (60) zwischen einem Abschnitt (56) der Leitungsmittel für plasmaarmes Blut und einem Abschnitt (58) der Plasmafiltrat-Leitungsmittel aufweisen, um den Plasmafiltratstrom durch die Plasmafiltrat-Leitungsmittel in Abhängigkeit von der Druckdifferenz zwischen den Leitungsmitteln für plasmaarmes Blut und den Plasmafiltrat-Leitungsmitteln einstellbar zu drosseln, so daß zwischen dem Druck des Plasmafiltrats und dem Druck des plasmaarmen Blutes ungeachtet zufälliger Veränderungen des Drucks des plasmaarmen Blutes im wesentlichen Gleichgewicht hergestellt und anschließend aufrechterhalten wird.

2. Membran-Plasmapheresevorrichtung nach Anspruch 1, wobei die Ausgleichsmittel einen ununterbrochenen Plasmafiltratstrom durch den Abschnitt (58) der Plasmafiltrat-Leitungsmittel aufrechterhalten.

3. Membran-Plasmapheresevorrichtung nach Anspruch 2, wobei die Ausgleichsmittel Mittel (60, 68, 62, 72, 76) aufweisen, die eine Laminarströmung durch den Abschnitt (58) der Plasmafiltrat-Leitungsmittel aufrechterhalten.

4. Membran-Plasmapheresevorrichtung nach Anspruch 1, 2 oder 3, wobei die Ausgleichsmittel eine ununterbrochene Strömung durch den Abschnitt (56) der Leitungsmittel für plasmaarmes Blut aufrechterhalten.

5. Membran-Plasmapheresevorrichtung nach Anspruch 4, wobei die Ausgleichsmittel Mittel (60, 62, 78, 80) aufweisen, die eine Laminarströmung durch den Abschnitt (56) der Leitungsmittel für plasmaarmes Blut aufrechterhalten.

6. Membran-Plasmapheresevorrichtung nach Anspruch 1, 2, 3, 4 oder 5, wobei die Ausgleichsmittel aufweisen: einen ersten, einen Teil der Plasmafiltrat-Leitungsmittel bildenden Leitungsabschnitt (54), einen zweiten, einen Teil der Leitungsmittel für plasmaarmes Blut bildenden Leitungsabschnitt (56), und einen dritten, mit dem ersten Leitungsabschnitt (54) in Verbindung stehenden Leitungsabschnitt (58), der eine flexible erste Wand (60) umfaßt, die die flexible Grenzfläche bildet und in Abhängigkeit von Fluiddrücken in den Plasmafiltrat-Leitungsmitteln und den Leitungsmitteln für plasmaarmes Blut wirksam ist und Fluidstrom zwischen dem ersten (54) und dem dritten (58) Leitungsabschnitt dosiert, um im wesentlichen das Gleichgewicht zwischen den Fluiddrücken in den Plasmafiltrat-Leitungsmitteln und den Leitungsmitteln für plasmaarmes Blut herzustellen und anschließend aufrechtzuerhalten.

7. Membran-Plasmapheresevorrichtung nach Anspruch 6, wobei der erste Leitungsabschnitt (54) eine erste Kammer bildet, der zweite Leitungsabschnitt (56) eine zweite Kammer bildet, die flexible erste Wand (60) eine Grenzfläche mit der zweiten Kammer bildet, und der dritte Leitungsabschnitt (58) eine zweite Wand (68) aufweist, die eine Grenzfläche mit der ersten Kammer bildet und eine eine Strömungsverbindung zwischen der ersten Kammer und dem dritten Leitungsabschnitt herstellende Öffnung (74) aufweist.

8. Membran-Plasmapheresevorrichtung nach Anspruch 7, wobei die erste Wand (60) im wesentlichen gegenüber der zweiten Wand (68) beabstandet ist und sich in Abhängigkeit von Schwankungen der Fluiddrücke in der zweiten Kammer (56) in einem Weg axial zur Öffnung (74) und von dieser weg bewegt zum Dosieren der Strömungsverbindung durch diese.

9. Membran-Plasmapheresevorrichtung nach Anspruch 8, wobei die flexible erste Wand (60) in Abhängigkeit von einem Anstieg der Fluiddrücke in der zweiten Kammer (56) in dem Weg zur Öffnung (74) bewegbar ist, um zunehmend die Strömungsverbindung durch diese zu drosseln, und wobei die flexible erste Wand (60) in Abhängigkeit von einer Abnahme der Fluiddrücke in der zweiten Kammer (56) in dem Weg von der Öffnung weg bewegbar ist, um die Drosselung der Strömungsverbindung zu verringern.

10. Membran-Plasmapheresevorrichtung nach Anspruch 9, wobei der Aufbau derart ist, daß ungeachtet der Dosierung durch die flexible erste Wand (60) eine kontinuierliche Strömungsverbindung zwischen den ersten (54) und dritten (58) Leitungsmitteln aufrechterhalten wird.

**Revendications**

1. Appareil de plasmaphérèse à membrane comprenant des moyens à membrane (20; 21) pour séparer le plasma du sang entier par filtrage; des moyens (22, 24) pour définir un passage de fluide, le long desdits moyens à membrane, présentant une chute de pression prédéterminée; des moyens (32) qui communiquant avec lesdits moyens à membrane et permettent la communication avec une source de sang entier, pour l'introduction du sang entier sous une pression d'entrée prédéterminée dans ledit passage de fluide afin de séparer le plasma du sang entier par filtrage; des moyens (34, 44, 56) qui communiquent avec lesdits moyens à membrane pour l'évacuation ou conduction du sang pauvre en plasma dudit passage de fluide, sous une pression de sang pauvre en plasma qui est sujette à des variations aléatoires; des moyens (36, 40, 54, 58) qui communiquent avec lesdits moyens à membrane pour l'évacuation ou conduction du filtrat de plasma dudit passage de fluide (24), sous une pression de filtrat de plasma; et des moyens d'égalisation (52) pour régler la chute de pression dans lesdits moyens à membrane et pour corriger la pression transmembrane, caractérisé en ce que les moyens d'égalisation comprennent une interface flexible (60), entre une partie (56) desdits moyens de conduction de sang pauvre en plasma et une partie (58) desdits moyens de conduction de filtrat de plasma, pour limiter de façon variable la circulation du filtrat de plasma dans lesdits moyens de conduction de filtrat de plasma, en réponse à la différence de pression entre lesdits moyens de conduction de sang pauvre en plasma et lesdits moyens de conduction de filtrat de plasma, afin d'établir et de maintenir ensuite un équilibre substantiel entre la pression du filtrat de plasma et la pression du sang pauvre en plasma, malgré les variations aléatoires de la pression du sang pauvre en plasma.

2. Appareil de plasmaphérèse à membrane suivant la revendication 1, dans lequel lesdits moyens d'égalisation maintiennent une circulation ininterrompue de filtrat de plasma dans ladite partie (58) des moyens de conduction de filtrat de plasma.

3. Appareil de plasmaphérèse à membrane suivant la revendication 2, dans lequel les moyens d'égalisation comprennent des moyens (60, 68, 62, 72, 76) pour maintenir un écoulement laminaire dans ladite partie (58) des moyens de conduction de filtrat de plasma.

4. Appareil de plasmaphérèse à membrane suivant la revendication 1, 2 ou 3, dans lequel lesdits moyens d'égalisation maintiennent une circulation ininterrompue dans ladite partie (56) des moyens de conduction de sang pauvre en plasma.

5. Appareil de plasmaphérèse à membrane suivant la revendication 4, dans lequel lesdits moyens d'égalisation comprennent des moyens (60, 62, 78, 80) pour maintenir un écoulement laminaire dans ladite partie (56) des moyens de conduction de sang pauvre en plasma.

6. Appareil de plasmaphérèse à membrane suivant la revendication 1, 2, 3, 4 ou 5, dans lequel lesdits moyens d'égalisation comprennent une première partie de conduit (54) constituant une partie desdits moyens de conduction du filtrat de plasma, une deuxième partie de conduit (56) constituant une partie desdits moyens de conduction de sang pauvre en plasma, et une troisième partie de conduit (58) communiquant avec ladite première partie de conduit (54) et comportant une première paroi flexible (60) qui constitue ladite interface flexible et qui fonctionne, en réponse aux pressions de fluide dans lesdits moyens de conduction de filtrat de plasma et dans lesdits moyens de conduction de sang pauvre en plasma, de manière à régler l'écoulement de fluide entre lesdites première (54) et troisième (58) parties de conduit pour établir et maintenir ensuite ledit équilibre substantiel entre les pressions de fluide dans lesdits moyens de conduction de filtrat de plasma et de sang pauvre en plasma.

7. Appareil de plasmaphérèse à membrane suivant la revendication 6, dans lequel ladite première partie de conduit (54) définit une première chambre, ladite deuxième partie de conduit (56) définit une deuxième chambre, la première paroi flexible (60) constitue une interface avec la deuxième chambre, et la troisième partie de conduit (58) comporte une deuxième paroi (68) formant une interface avec la première chambre et comportant un orifice (74) qui établit une communication d'écoulement entre la première chambre et la troisième partie de conduit.

8. Appareil de plasmaphérèse à membrane suivant la revendication 7, dans lequel la première paroi (60) est sensiblement en face et espacée de la deuxième paroi (68) et fonctionne de manière à se déplacer, en réponse à des fluctuations des pressions de fluide dans ladite deuxième chambre (56), suivant un chemin axial pour se rapprocher et s'éloigner dudit orifice (74) afin de régler la communication d'écoulement à travers cet orifice.

9. Appareil de plasmaphérèse à membrane suivant la revendication 8, dans lequel, en réponse à une augmentation des pressions de fluide dans ladite deuxième chambre (56), la première paroi flexible (60) se déplace suivant ledit chemin vers ledit orifice (74) de manière à réduire davantage la communication d'écoulement à travers cet orifice, et dans lequel, en réponse à une diminution desdites pressions de fluide dans la deuxième chambre (56), la première paroi flexible (60) se déplace suivant ledit chemin de manière à s'éloigner dudit orifice pour réduire la limitation de ladite communication d'écoulement.

10. Appareil de plasmaphérèse à membrane suivant la revendication 9, dans lequel la construction est telle qu'une communication d'écoulement continu entre les première (54) et troisième (58) parties de conduit est maintenue, indépendamment du réglage par la première paroi flexible (60).

0 082 187

FIG. 1

FIG. 2

## FIG. 3

SOURCE OF
PRESSURIZED
FLUID

SOURCE OF
PRESSURIZED
FLUID

## FIG. 4

## FIG. 5

SOURCE

SOURCE

# FIG. 6